# EUROPEAN PATENT APPLICATION

(11) **EP 4 686 490 A1**
(43) Date of publication of application: **04.02.2026**
(21) Application number: 25185578.9
(22) Date of filing: 26.06.2025
(51) Int. Cl.: A61M 13/00

(54) **MEDICAL DEVICE FOR PIPAC**

(30) Priority: 30.07.2024 IT 202400017677
(71) Applicant: AC. TA. S.R.L., 80045 Pompei, Napoli (IT)
(72) Inventor: DI CECIO, Mario, 80045 Pompei, Napoli (IT); VAIRA, Marco, 80045 Pompei, Napoli (IT)
(74) Representative: Gualeni, Nadia

(57) **Abstract**

A medical device (1) for a pressurized intraperitoneal aerosolized chemotherapy (PIPAC) procedure according to the present invention comprises a cannula (2), connected to a handle (3), and includes at least one nozzle lumen (23) for a slidable nozzle (5) for aerosol nebulization, and at least one evacuation lumen (24). The nozzle (5) comprises a nozzle body (51) ending distally in a nozzle head (52) with a nebulization opening (521), and is provided internally with at least one drug lumen (510) for drug injection and at least one gas lumen (511) for gas injection. The nozzle head (52) includes an aerosol chamber (6) into which the at least one drug lumen (510) and the at least one gas lumen (511) discharge for aerosol formation. Such a medical device enables optimal drug distribution in the peritoneal cavity.

## Description

The subject of the present invention is a medical device for performing a pressurized intraperitoneal aerosolized chemotherapy procedure.

Pressurized intraperitoneal aerosolized chemotherapy, known as PIPAC (Pressurized IntraPeritoneal Aerosolized Chemotherapy), is an experimental loco-regional oncological treatment, meaning it allows for the execution of a chemotherapeutic regimen in a specific compartment of the body (in this case the peritoneum of patients affected by peritoneal carcinomatosis). The rationale of this procedure combines two principles: ensuring effective distribution of chemotherapeutic drugs within the selected compartment, while strongly limiting the systemic diffusion of the drugs themselves, resulting in a reduction of the systemic impact of chemotherapy.

PIPAC is a recently introduced technique, developed for the treatment of peritoneal carcinomatosis in patients who cannot undergo cytoreductive surgery. The technique allows drug administration via aerosol, using a laparoscopic access, as well as the execution of biopsies for histological study and the aspiration of ascitic fluid if present.

PIPAC is a technically simple procedure and lasts 30 minutes. It initially involves increasing the pressure to 12 mmHg and the temperature to 37°C within the patient's peritoneal cavity; subsequently, a cytotoxic solution in aerosol form (i.e., chemotherapeutic drug particles dispersed in gas, generally carbon dioxide) is nebulized in the abdominal cavity for 30 minutes; the aerosol is then removed through a closed suction system.

In PIPAC, the artificially generated pressure gradient with which the cytotoxic solution is nebulized exceeds the pressure of the tumor interstitial fluids, which represent an obstacle in conventional antitumor therapy. Furthermore, through PIPAC, the local concentration of the chemotherapeutic drug is much higher than what would be achieved via intravenous administration, but the concentration of the chemotherapeutic drug in the plasma remains low. With PIPAC, only 10% of the chemotherapeutic dosage normally used systemically in other loco-regional treatments like HIPEC (hyperthermic intraperitoneal chemotherapy) is applied, with the great advantage that typical side effects of systemic chemotherapy (alopecia, peripheral neurotoxicity, cardiotoxicity, nausea, myelosuppression) are generally not observed or are mild. For this reason, PIPAC is a promising method, capable of inducing regression of peritoneal carcinomatosis even in advanced stages and in conditions seemingly unresponsive to systemic therapy, with limited side effects.

Conventional PIPAC involves multi-port access, meaning 2/3 peritoneal accesses are used, as in the state-of-the-art solution described in document EP 4 000 672. Considering that patients with carcinomatosis have often undergone previous surgeries and present many visceroparietal adhesions, traditional access more easily exposes the patient to the risk of visceral and often unrecognized iatrogenic injuries. Furthermore, such traditional access requires the placement of 2/3 trocars in different abdominal locations, with a non-negligible risk of neoplastic seeding along the trocar paths themselves. An example of a known device for PIPAC procedures is described in document DE 10 2012 104629 A1: such a device lacks distinct lumens for drug and gas, and thus lacks an internal chamber for aerosol formation, since the drug arrives already pressurized at the delivery head. This solution is disadvantageous because it requires a complex pressurization system external to the device. Other known devices in the field include those for introducing a drug into a body cavity, such as described in WO 2023/161419 A1, or for nebulizing an aerosol, such as described in US 2008/283048 A1, which however are not suitable for optimally treating the abdominal cavity of a patient undergoing the PIPAC procedure.

A recently conceived method involves a single peritoneal access (single-port access), which allows reducing or even eliminating the negative aspects mentioned above regarding traditional multi-port access. With the single-port, access to the peritoneal cavity is unique and involves a wider opening (5 cm incision) made along the midline. These characteristics allow access to the peritoneal cavity under direct vision due to the wider incision, and the execution of adhesiolysis while minimizing the risk of iatrogenic injuries. Furthermore, in case of seeding along the access path, this is located on the midline and can therefore be more easily removed in subsequent laparotomies. However, to date there are few and inefficient medical devices for performing PIPAC procedures with single-port access.

The object of the present invention is to provide a medical device for performing a PIPAC procedure with single-port access that allows optimal drug distribution in the peritoneal cavity.

This object is achieved by a medical device in accordance with claim 1. The dependent claims describe preferred embodiments of the invention.

The features and advantages of a medical device for performing PIPAC procedures with single-port access will be evident from the description given below, provided by way of example and not limitation, in accordance with the attached figures, in which:
- Figure 1 shows an axonometric view of a medical device according to the present invention, in a first embodiment, in the closed configuration of the nozzles;
- Figure 2 shows an axonometric view of the medical device of Figure 1, in the open configuration of the nozzles;
- Figures 3A and 3B show an enlargement of the distal area of the medical device of Figure 1 and Figure 2, respectively;
- Figure 4 shows a side view with the distal portion partially in section of the medical device of Figure 1;
- Figure 5 shows a side view of the medical device of Figure 1 in which, for clarity of illustration, the handle has been removed and the central and distal portion is partially in section;
- Figure 6 shows a side view of the medical device of Figure 2 in which, for clarity of illustration, the central and distal portion is partially in section;
- Figure 7 shows an enlargement of the distal portion of the medical device of Figure 4;
- Figure 8 shows a sectional view of the cannula of the medical device of Figure 4;
- Figure 9 shows a partially transparent axonometric view of a nozzle of the medical device according to the present invention;
- Figures 10A to 10C show some details of the nozzle of Figure 9, and in particular Figure 10A shows an internal view toward the distal outlet, Figure 10B shows a lateral sectional view, Figure 10C shows a partial section;
- Figure 11 shows a side view with the distal portion partially in section of a medical device according to the present invention, in a second embodiment, in the closed configuration of the nozzles;
- Figure 12 shows a side view of the medical device of Figure 11, in the open configuration of the nozzles;
- Figure 13 shows an axonometric view of the medical device of Figure 12;
- Figure 14 shows an enlargement of the distal portion of the medical device of Figure 11;
- Figure 15 shows a sectional view of the cannula of the medical device of Figure 11.

With reference to the attached figures, the overall reference number 1 denotes a medical device for performing a pressurized intraperitoneal aerosolized chemotherapy (PIPAC) procedure in accordance with the present invention. The medical device 1 is suitable for nebulizing an aerosol in the abdominal cavity of a patient to be treated using the PIPAC procedure. The aerosol is a liquid nebulized in a gaseous medium, and in this specific case it is a chemotherapeutic drug dispersed in a gas (e.g., nitrogen, carbon dioxide).

In the following discussion, with the physician performing the PIPAC procedure conventionally established as the central point of reference, the terms proximal (i.e., closer to the physician) and distal (i.e., farther from the physician) are defined.

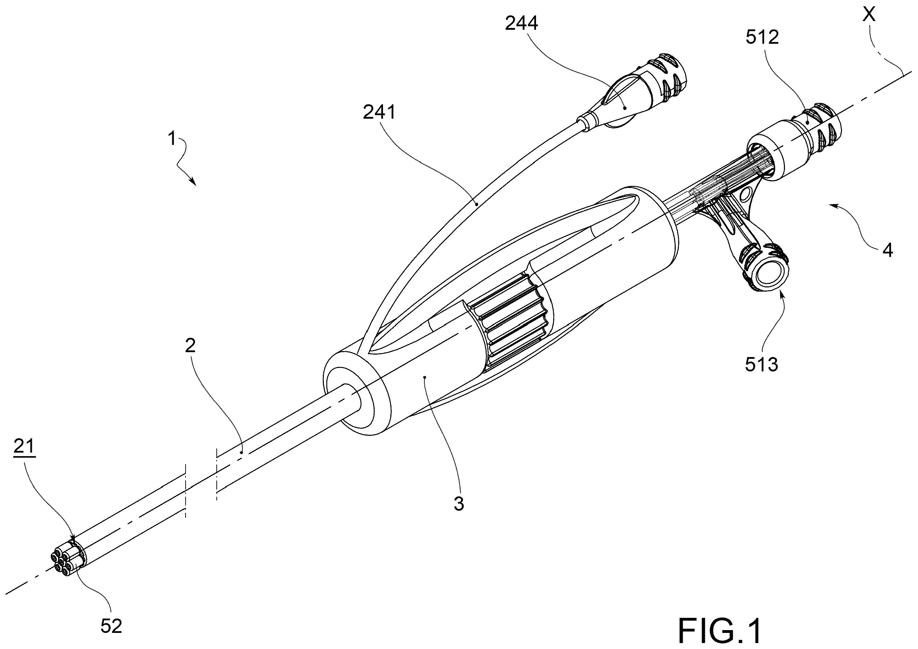

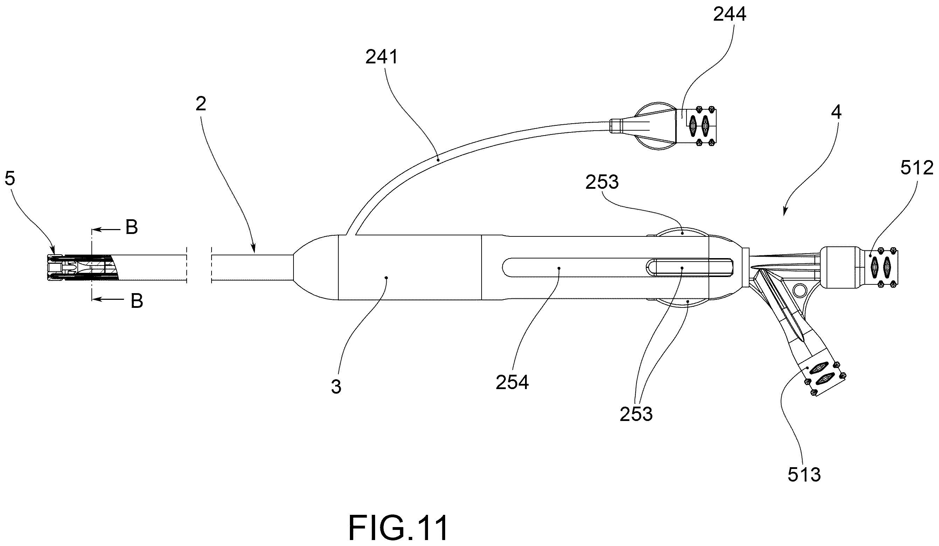

The medical device 1 therefore comprises a distal portion, intended to be at least partially inserted into the abdominal cavity of a patient, a central area, and a proximal area closer to the physician. According to this frame of reference, the medical device 1 comprises distally a cannula 2, centrally a handle 3, and proximally a connector area 4, as shown in Figures 1 and 11.

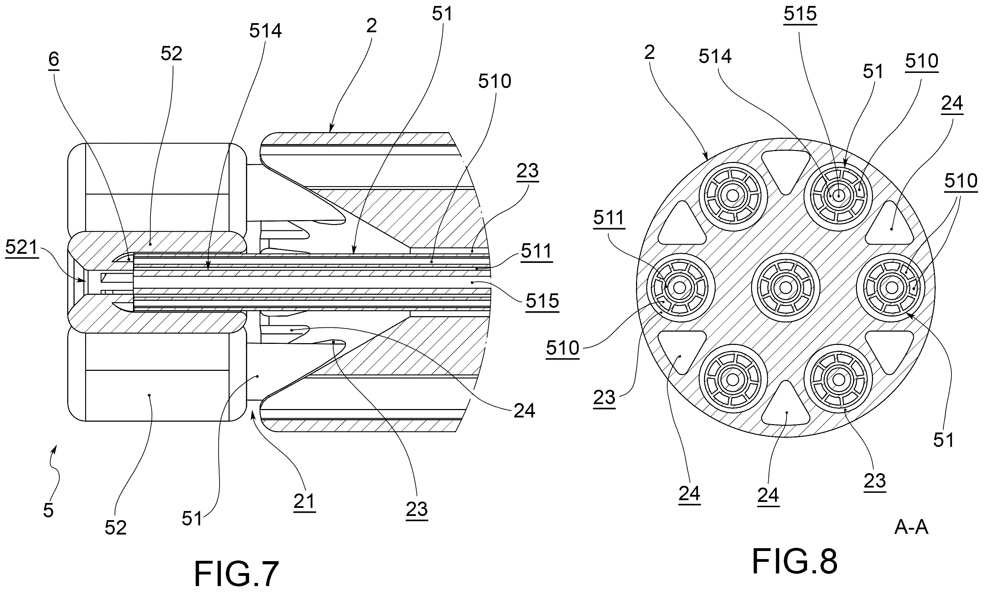

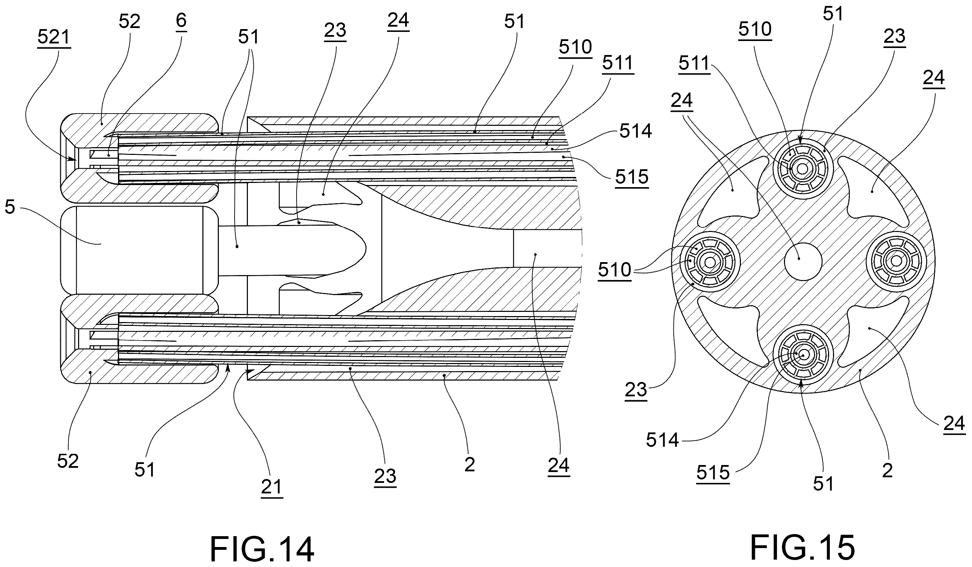

The cannula 2 extends along a longitudinal axis X and is provided with a cylindrical body with a plurality of lumens, or channels, extending within it, as clearly visible in the sections of Figures 8 and 15. Preferably, the cannula 2 has a circular cross-section.

The cannula 2 extends between a distal outlet 21 and a proximal inlet 22. The internal lumens of the cannula 2 extend between the distal outlet 21 and the proximal inlet 22.

The cannula 2 comprises at least one nozzle lumen 23 intended to slidingly house a nozzle 5 for nebulizing the aerosol within the abdominal cavity of a patient undergoing PIPAC. Preferably, the nozzle lumen 23 has a circular cross-section.

In the case of a single nozzle lumen 23, it is preferably arranged centrally within the cannula 2.

Preferably, the cannula 2 comprises a plurality of nozzle lumens 23 in order to house a plurality of nozzles 5 for more effective aerosol nebulization. Preferably, the medical device 1 comprises at least four nozzles, for example six or seven nozzles.

In the case of multiple nozzle lumens 23, they are uniformly distributed, for example forming a circular crown, or arranged in a line.

The cannula comprises at least one evacuation lumen 24 configured to allow the evacuation, at the end of the PIPAC procedure, of the gas and residual chemotherapeutic drug. Preferably, evacuation takes place through a closed system connected to the medical device 1, which prevents environmental dispersion of the antiblastic drug.

The evacuation lumen 24 is connected to an evacuation conduit 241 that partially protrudes from the handle 3 and ends in an evacuation connector 244.

Preferably, the cannula 2 comprises a plurality of evacuation lumens 24 for more effective evacuation of the aerosol and residual gases at the end of the procedure. In the case of multiple evacuation lumens 24, they are uniformly distributed, for example forming a circular crown.

Figures 8 and 15 show exemplary embodiments of a multi-lumen cannula 2, equipped with a plurality of nozzle lumens 23 uniformly distributed to form a circular crown, in alternating sequence with a plurality of evacuation lumens 24, also uniformly distributed to form a circular crown.

In the case of a multi-lumen cannula 2, to optimize the use of available space, a central lumen may also be provided, intended to be an evacuation lumen (as in Figure 15) or a nozzle lumen (as in Figure 8).

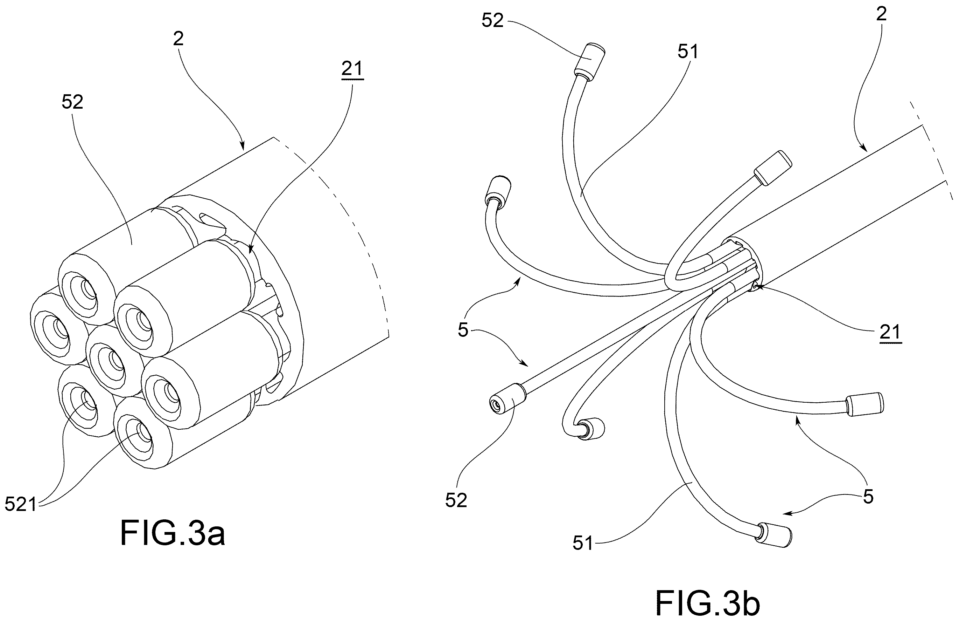

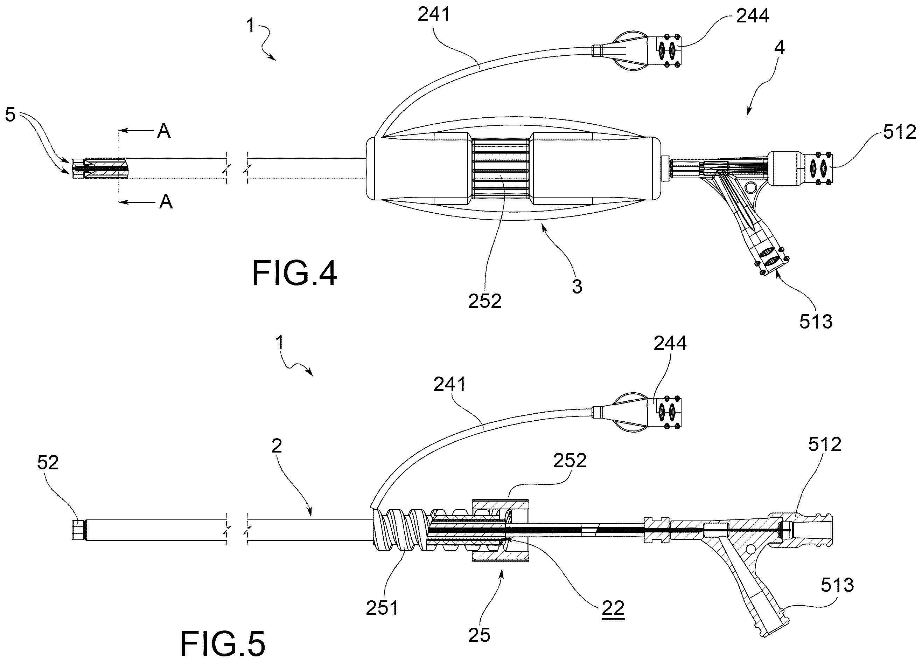

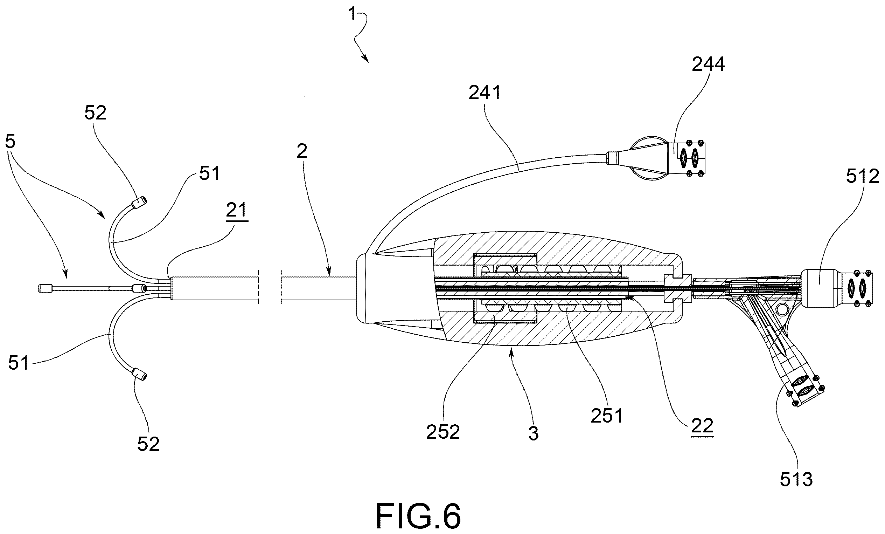

In the example of Figures 1 to 10, the cannula 2 is fixed to the handle 3 and is slidable with respect to said handle 3 by movement means 25. In this embodiment, retraction of the cannula 2 allows exposure of the nozzle 5 in the case of a single-nozzle medical device 1, or simultaneous exposure (Figure 3B) or retraction (Figure 3A) of the nozzles 5 in the case of a multi-nozzle medical device 1. The movement means, shown in detail in Figures 5 and 6, comprise a worm gear mechanism, formed by a screw 251 slidingly inserted into a nut 252. The screw 251 is hollow inside to house the cannula 2 with which it is integral. The nut 252 is internally hollow to slidingly house the screw 251. The nut 252 forms an actuation wheel, protruding externally from the handle 3 to allow actuation of the movement means 25.

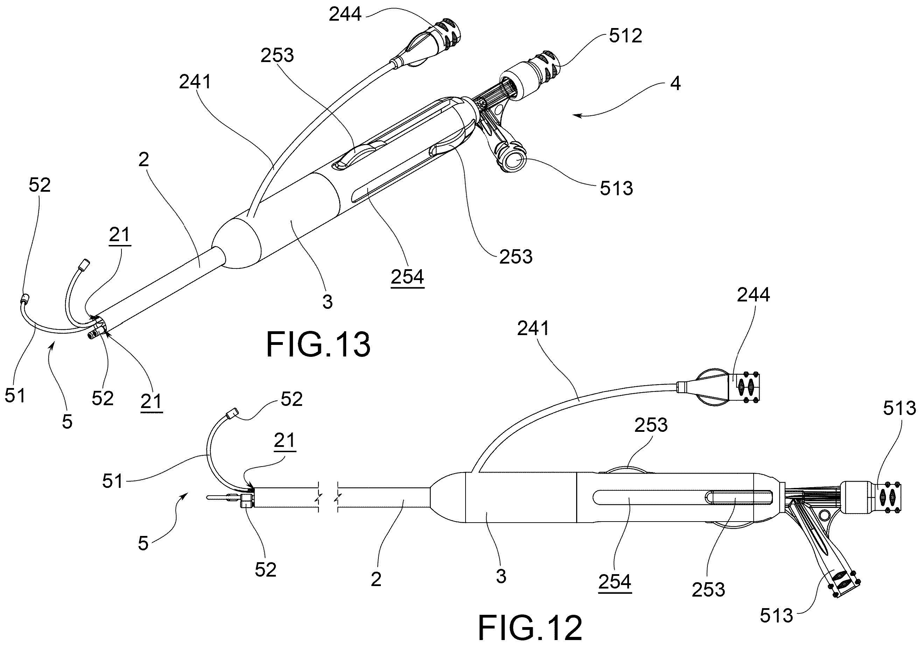

In the example of Figures 11 to 15, the cannula 2 is integral with the handle 3 and it is the nozzle 5 in the case of a single-nozzle medical device 1, or the nozzles 5 in the case of a multi-nozzle medical device 1, that are slidable for selective advancement and retraction (Figures 12 and 13). The movement means comprise a selector 253, integral with the nozzle 5 and protruding at least partially from the handle, slidable in a track 254 made in the handle 3.

Preferably, the handle 3 at least partially covers the movement means, facilitating the grip of the medical device 1.

The medical device 1 comprises at least one nozzle 5 for aerosol nebulization inside the abdominal cavities of a patient to be treated using PIPAC. The nozzle 5 comprises a nozzle body 51 ending distally in a nozzle head 52. The nozzle head 52 protrudes from the distal outlet 21 of the cannula 2.

The nozzle body 51 is tubular and multi-lumen, i.e., equipped with a plurality of lumens or channels that extend within it, as clearly shown in the sections of Figures 8 and 15.

The nozzle body 51 comprises at least one drug lumen 510 intended for the injection of the chemotherapeutic drug that will form, within the nozzle head 52, the aerosol to be nebulized inside the patient's abdominal cavity. The drug lumen 510 is connected to a drug conduit that ends in a drug connector 512. The drug connector 512 is connected, for example, to a dosing syringe.

Preferably, the nozzle body 51 comprises a plurality of drug lumens 510 for more effective drug dosing. In the case of multiple drug lumens 510, they are uniformly distributed, for example forming a circular crown.

The nozzle body 51 also comprises at least one gas lumen 511 intended for the injection of a gas that will form, within the nozzle head 52, the aerosol to be nebulized inside the patient's abdominal cavity.

The gas lumen 511 is connected to a gas conduit that ends in a gas connector 513. The gas connector 513 is connected, for example, to a gas cylinder (e.g., CO₂ or nitrogen), or to the mechanical ventilation system of the operating room, or to a dedicated device (such as a portable aerosol machine).

In a preferred embodiment, the nozzle body 51 is made of flexible plastic material. This solution allows the nozzle body 51 to be shaped into the desired curvature.

Preferably, the gas lumen 511 of the nozzle body 51 houses a shape-memory stem 514 having a certain curvature, for example a C-shape. The stem 514 imparts the desired curvature to the nozzle body 51.

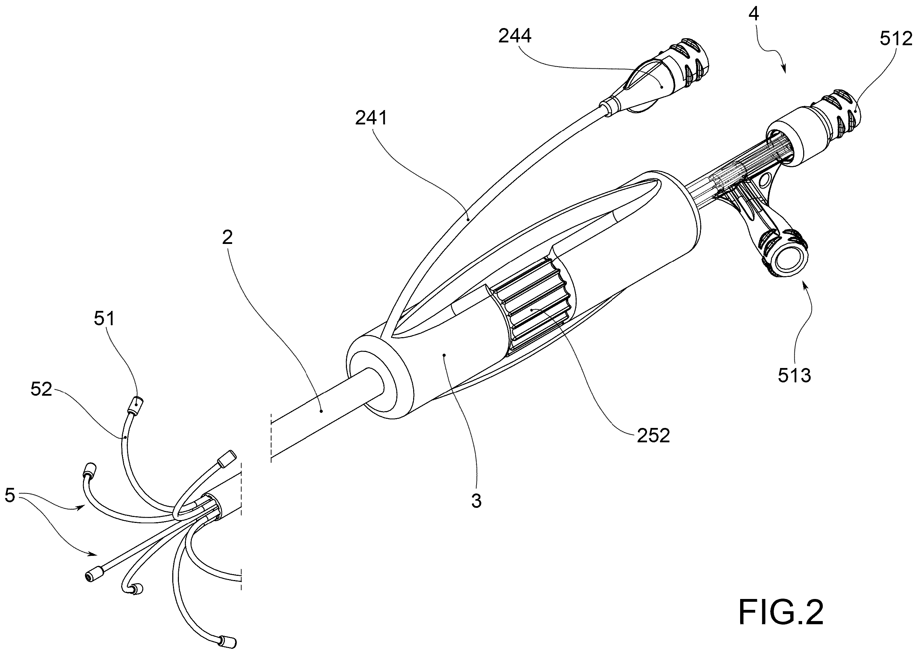

For clarity, it is noted that the nozzle body 51 is more flexible than the stem 514 and therefore conforms to it. At the same time, the cannula 2 is rigid, and when the stem 514 is inserted inside it, it is forced to straighten. When the nozzle 5 is released from the cannula 2 (by retracting the cannula 2 as in Figure 2, or by selective advancement of the nozzle 5 as in Figure 13), the flexible nozzle body 51 adapts to the curvature of the stem 514. In the case of a multi-nozzle medical device 1, the distribution of nozzles 5 can be optimized by forming, for example, a flower or umbrella configuration (Figure 3B), thereby achieving optimal and uniform aerosol distribution.

Moreover, by controlling the degree of release of the nozzle 5, it is possible to manage the degree or radius of curvature of the nozzle body 51: partial release results in partial curvature, full release results in full curvature.

In one embodiment, the stem 514 is provided with a gas sub-lumen 515 intended for injecting a gas that will form, within the nozzle head 52, the aerosol to be nebulized inside the patient's abdominal cavity. The gas sub-lumen 515 is connected to a gas conduit that ends in a gas connector 513. In this example, the gas lumen 511 of the nozzle body 51 is used solely to house the stem 514.

As mentioned above, the aerosol to be nebulized inside the patient's abdominal cavity is formed within the nozzle head 52. Indeed, as shown in Figures 7 and 14, the drug and the gas transported by the nozzle 5 meet within the nozzle head 52.

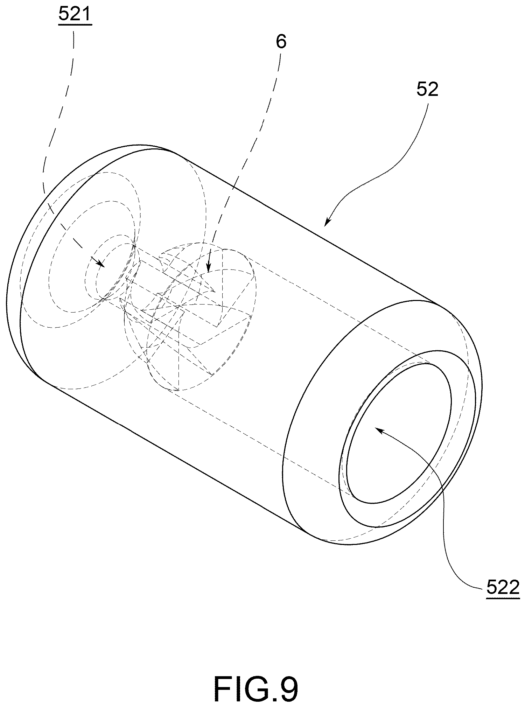

The nozzle head 52 includes (shown in detail in Figures 9 to 10C), from the distal to the proximal direction, a nebulization opening 521, an aerosol chamber 6, and a nozzle chamber 522.

The distal end of the nozzle body 5 is positioned and fixed within the nozzle chamber 522. The drug lumen 510 and the gas lumen 511 discharge into the aerosol chamber 6, where the aerosol is formed. The aerosol exits through the nebulization opening 521.

The aerosol chamber 6 is an expansion chamber where the gas, subjected to a vortex effect, meets the drug, resulting in its fine fragmentation.

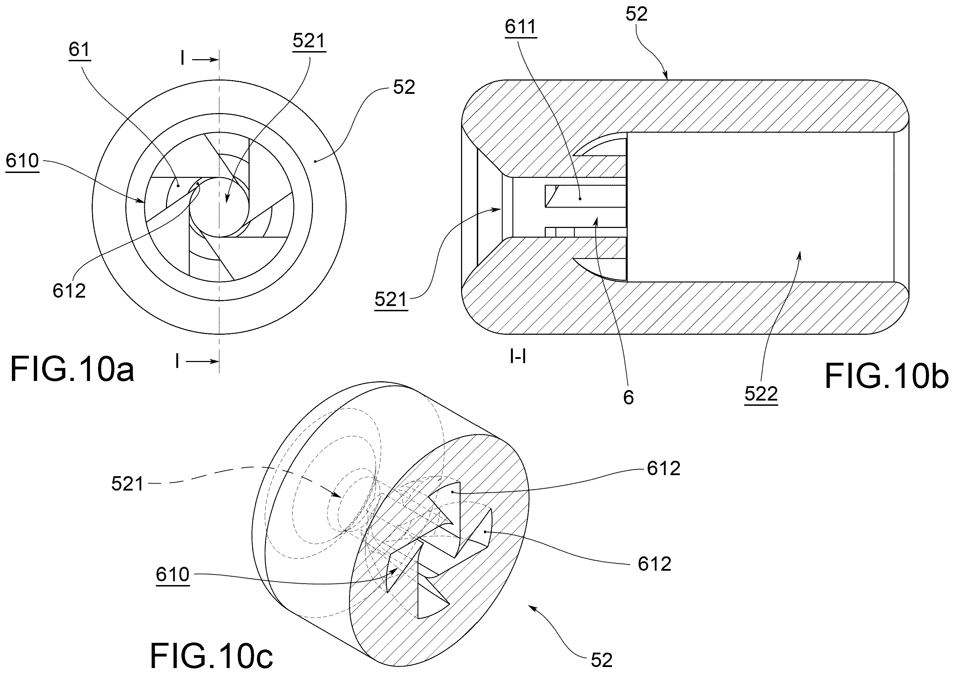

The aerosol chamber 6 is formed by a plurality of conical micro-conduits 61 (e.g., four in Figure 10C). In the micro-conduit 61, the gas and the drug undergo a directional change, from axial to transverse. The micro-conduit 61 features an axial inlet 610 (e.g., substantially triangular) and a transverse outlet 611 facing inward, i.e., toward the longitudinal axis X (e.g., substantially rectangular). Between the inlet and the outlet, the micro-conduit 6 has a curved wall 612.

An example of how to use the medical device for performing a PIPAC procedure with single-port access according to the present invention will now be described.

The patient, in a supine position and under general anesthesia, is subjected to a ~5 cm incision along the midline to ensure an "open" access to the peritoneal cavity, minimizing the risk of intestinal injuries upon entry inside said cavity. Once access to the peritoneal cavity is achieved and if conditions are appropriate to proceed with the intervention, a single-port device is applied and pneumoperitoneum is induced at 12 mmHg. The peritoneal cavity is then explored: if ascites is present, it is aspirated; the extent and characteristics of peritoneal carcinomatosis are measured; biopsies are taken for histological analysis. At this point, the medical device 1 is introduced into the patient's abdominal cavity through the single-port device, and it is verified that there is no gas leakage from the access site to the abdomen. After correct positioning/exposure of the nozzles 5, nebulization of the cytostatic drug and gas mixture (aerosol) is carried out to aerosolize the chemotherapeutic drug. Insufflation lasts 30 minutes. After this time, the gas and residual chemotherapeutic drug are aspirated through a so-called "closed system" to prevent environmental dispersion of the antiblastic drug. Once the nozzles 5 are retracted into the cannula 2, the medical device 1 is removed from the peritoneal cavity. Pneumoperitoneum is then re-induced, hemostasis is verified, the single-port used for insufflation is removed, and the peritoneal access is sutured. Normally, no abdominal drains or nasogastric tubes are placed.

Innovatively, a medical device for performing a PIPAC procedure with single-port access according to the present invention enables optimal distribution of the drug within the peritoneal cavity.

Advantageously, in the case of a multi-nozzle medical device, a flower or umbrella configuration can be achieved, allowing optimal and uniform aerosol distribution.

Advantageously, in the case of a medical device with selective advancement of the nozzle or nozzles, dedicated aerosol nebulization in a specific treatment area is possible.

Advantageously, the medical device 1 is equipped with an aerosol chamber 6 which is an expansion chamber specifically designed to achieve fine fragmentation of the drug and optimal aerosolization.

It is clear that a person skilled in the art may make modifications to the medical device described above, all of which fall within the scope of protection as defined by the following claims.

## Claims

1. A medical device (1) for a pressurized intraperitoneal aerosolized chemotherapy procedure (PIPAC) comprising:
- a cannula (2) extending along a longitudinal axis (X) and connected to a handle (3); wherein said cannula (2) comprises at least one nozzle lumen (23) therein, intended to slidingly house a nozzle (5) for nebulizing an aerosol, and preferably at least one evacuation lumen (24);
- at least one nozzle (5) for nebulizing an aerosol; wherein said nozzle (5) comprises a nozzle body (51) ending distally in a nozzle head (52) with a nebulization opening (521); wherein said nozzle body (51) comprises therein at least one drug lumen (510) for injecting a drug and at least one gas lumen (511) for injecting a gas;
wherein said nozzle head (52) comprises an aerosol chamber (6) in which the at least one drug lumen (510) and the at least one gas lumen (511) flow for the formation of aerosol.

2. Medical device (1) according to claim 1, wherein said aerosol chamber (6) is formed by a plurality of micro-conduits (61), each micro-conduit (61) being provided with an axial inlet (610), at which the at least one drug lumen (510) and the at least one gas lumen (511) flow, and with a transverse outlet (611).

3. Medical device (1) according to claim 2, wherein said outlet (611) of the micro-conduit (6) faces the longitudinal axis (X).

4. Medical device (1) according to claim 2 or 3, wherein said micro-conduit (6) has a curved wall (612) between the inlet (610) and the outlet (611).

5. Medical device (1) according to any one of the preceding claims, wherein the cannula (2) comprises a plurality of nozzle lumens (23), each housing a nozzle (5).

6. Medical device (1) according to any one of the preceding claims, wherein the cannula (2) comprises at least four nozzle lumens (23), each housing a nozzle (5), said nozzle lumens (23) being evenly distributed to form a circular crown or being arranged in line.

7. Medical device (1) according to any one of the preceding claims, wherein the cannula (2) comprises a plurality of evacuation lumens (24).

8. Medical device (1) according to any one of the preceding claims, wherein the nozzle body (51) comprises a plurality of evenly distributed drug lumens (510).

9. Medical device (1) according to any one of the preceding claims, wherein the gas lumen (511) of the nozzle body (51) houses a stem (514), said stem (514) having shape-memory and being provided with a curvature, and wherein the nozzle body (51) is flexible and the stem (514) imparts a curvature to the nozzle body (51).

10. Medical device (1) according to claim 9, wherein the stem (514) is provided with a gas sub-lumen (515) for injecting a gas.

11. Medical device (1) according to any one of the preceding claims, wherein the cannula (2) slides with respect to the handle (3) through movement means (25), and wherein the retraction of the cannula (2) allows for the exposure of the at least one nozzle (5).

12. Medical device (1) according to claim 11, wherein said movement means comprise a screw (251) integral with the cannula (2) and slidingly inserted into a nut (252), wherein said nut (252) forms an actuation wheel protruding outside the handle (3).

13. Medical device (1) according to any one of claims 1 to 10, wherein the cannula (2) is integral with the handle (3) and said at least one nozzle (5) slides with respect to the cannula (2) through movement means.

14. Medical device (1) according to claim 13, wherein said movement means comprise a selector (253) integral with the nozzle (5) and protruding at least partially from the handle, said selector (253) being slidable in a track (254) obtained in the handle (3).
